# EUROPEAN PATENT SPECIFICATION

(11) **EP 2 852 417 B1**
(45) Date of publication and mention of the grant of the patent: **12.06.2019**
(21) Application number: 13714066.1
(22) Date of filing: 11.03.2013
(51) Int. Cl.: A61L 27/44, A61L 27/46, A61L 27/58, A61L 31/12, A61L 31/14, C08L 67/04

(54) **RESORBABLE BIOCERAMIC COMPOSITIONS OF POLY-4-HYDROXYBUTYRATE AND COPOLYMERS**
RESORBIERBARE BIOKERAMISCHE ZUSAMMENSETZUNGEN AUS POLY-4-HYDROXYBUTYRAT UND COPOLYMEREN
COMPOSITIONS DE BIOCÉRAMIQUE RÉSORBABLE DE POLY-4-HYDROXYBUTYRATE ET LEURS COPOLYMÈRES

(30) Priority: 21.05.2012 US 201261649506 P; 12.10.2012 US 201261713139 P
(43) Date of publication of application: 01.04.2015
(60) Divisional application: 17174070.7
(73) Proprietor: Tepha, Inc., Lexington, MA 02421 (US)
(72) Inventor: CARTER, Andrew, J., Stow, MA 01775 (US); RIZK, Said, Windham, NH 03087 (US); MARTIN, David, P., Arlington, MA 02474 (US)
(74) Representative: Potter Clarkson
(86) International application number: PCT/US2013/030177
(87) International publication number: WO 2013/176734

(56) References cited:
- EP-A1- 0 714 666
- WO-A1-00/18443
- WO-A1-2006/015316
- WO-A1-2008/095083
- WO-A1-2009/085823
- WO-A1-2010/129882
- WO-A2-2007/140325
- US-A- 5 697 980
- US-A1- 2012 290 073
- US-B1- 6 610 764
- DATABASE WPI Week 199317 Thomson Scientific, London, GB; AN 1993-137113 XP002698634, & JP H05 70696 A (TOPPAN PRINTING CO LTD) 23 March 1993 (1993-03-23) -& DATABASE EPODOC [Online] EUROPEAN PATENT OFFICE, THE HAGUE, NL; XP002698635, & JP H05 70696 A (TOPPAN PRINTING CO LTD) 23 March 1993 (1993-03-23)
- C.DOYLE ET AL.: "In vitro and in vivo evaluation of polyhydroxybutyrate and of polyhydroxybutyrate reinforced with hydroxyapatite", BIOMATERIALS, vol. 12, November 1991 (1991-11), pages 841-847, XP002698636,
- KNOWLES J C ET AL: "Development of a degradable composite for orthopaedic use: in vivo biomechanical and histological evaluation of two bioactive degradable composites based on the polyhydroxybutyrate polymer", BIOMATERIALS, ELSEVIER SCIENCE PUBLISHERS BV., BARKING, GB, vol. 13, no. 8, 1992, pages 491-496, XP024142678, ISSN: 0142-9612, DOI: 10.1016/0142-9612(92)90099-A [retrieved on 1992-01-01]
- "Advances in Biomaterials" In: P.J.Doherty et al. (Eds.): "Biomaterial-Tissue Interfaces", 1992, Elsevier Science Publishers, XP8162921, pages 439-446, the whole document
- S.K.MISRA ET AL.: "Polyhydroxyalkanoate (PHA)/Inorganic Phase Composites for Tissue Engineering Applications", BIOMACROMOLECULES, vol. 7, no. 8, August 2006 (2006-08), pages 2249-2258, XP002698656,
- MARTIN D P ET AL: "Medical applications of poly-4-hydroxybutyrate: A strong flexible absorbable biomaterial", BIOCHEMICAL ENGINEERING JOURNAL, ELSEVIER, AMSTERDAM, NL, vol. 16, no. 2, 2003, pages 97-105, XP002530911, ISSN: 1369-703X, DOI: 10.1016/S1369-703X(03)00040-8

## Description

### CROSS REFERENCE TO RELATED APPLICATIONS

### FIELD OF THE INVENTION

The present invention generally relates to medical devices comprising resorbable bioceramics and poly-4-hydroxybutyrate and copolymers thereof. The compositions can be used in many types of implant applications including orthopedic, craniomaxillofacial, and dental applications, as well as in oral surgery, plastic and reconstructive surgery, ear, nose and throat surgery, and general surgery.

### BACKGROUND OF THE INVENTION

Poly-4-hydroxybutyrate (P4HB) and copolymers thereof can be produced using transgenic fermentation methods, see, for example, U.S. Patent No. 6,548,569 to Williams et al.*,* and are produced commercially, for example, by Tepha, Inc. (Lexington, MA). Poly-4-hydroxybutyrate (P4HB, TephaFLEX® biomaterial) is a strong, pliable thermoplastic polyester that, despite its biosynthetic route, has a relatively simple structure.

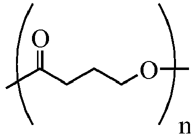

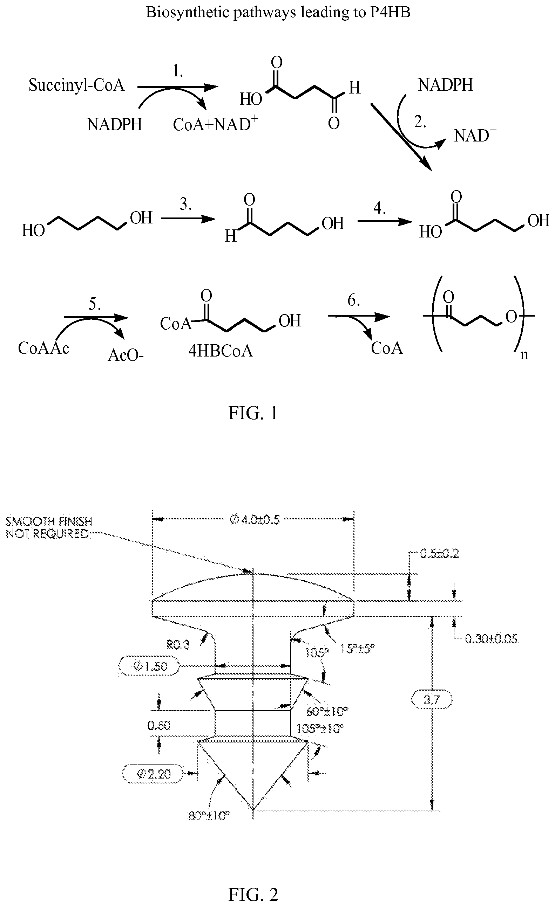

The polymer belongs to a larger class of materials called polyhydroxyalkanoates (PHAs) that are produced by numerous microorganisms (see, for example, Steinbüchel A., et al. Diversity of Bacterial Polyhydroxyalkanoic Acids, FEMS Microbial. Lett. 128:219-228 (1995)). In nature these polyesters are produced as storage granules inside cells, and serve to regulate energy metabolism. They are also of commercial interest because of their thermoplastic properties, biodegradability and relative ease of production. Several biosynthetic routes are currently known to produce P4HB as shown in Figure 1.

Chemical synthesis of P4HB has been attempted, but it has been impossible to produce the polymer with a sufficiently high molecular weight that is necessary for most applications (see Hori, et al., Polymer 36:4703-4705 (1995); Houk, et al., J. Org. Chem., 73 (7):2674-2678 (2008); and Moore, T., et al., Biomaterials 26:3771-3782 (2005)). In fact, it has been calculated to be thermodynamically impossible to chemically synthesize a high molecular weight homopolymer under normal conditions (Moore, et al., Biomaterials 26:3771-3782 (2005)). Examples of high molecular weight P4HB and copolymers thereof with weight average molecular weights in the region of 50,000 to 1,000,000 Da are produced by Tepha, Inc. of Cambridge, MA, using transgenic fermentation methods.

U.S. Patents Nos. 6,245,537, 6,623,748 and 7,244,442 describe methods of making PHAs with little to no endotoxin, which are suitable for medical applications. U.S. Patent Nos. 6,548,569, 6,838,493, 6,867,247, 7,268,205, 7,179,883, 7,943,683, WO 09/085823 to Ho et al.*,* and WO 11/159784 to Cahil et al. describe the use of PHAs to make medical devices. Copolymers of P4HB including 4-hydroxybutyrate copolymerized with 3-hydroxybutyrate or glycolic acid are described in U.S. patent application No. 20030211131 by Martin and Skraly, U.S. Patent No. 6,316,262 to Huisman et al.*,* and U.S. Patent No. 6,323,010 to Skraly et al. Methods to control the molecular weight of PHA polymers produced by biosynthetic methods have been disclosed by U.S. Patent No. 5,811,272 to Snell et al.

PHAs with controlled degradation and degradation *in vivo* of less than one year are disclosed by U.S. Patent No. 6,548,569, 6,610,764, 6,828,357, 6,867,248, and 6,878,758 to Williams et al. and WO 99/32536 to Martin et al. Applications of P4HB have been reviewed in Williams, S.F., et al., Polyesters, III, 4:91-127 (2002), and by Martin, D. et al. Medical Applications of Poly-4-hydroxybutyrate: A Strong Flexible Absorbable Biomaterial, Biochem. Eng. J. 16:97-105 (2003). Medical devices and applications of P4HB have also been disclosed by WO 00/56376 to Williams et al. Several patents including U.S. Patent Nos. 6,555,123, 6,585,994, and 7,025,980 describe the use of PHAs in tissue repair and engineering.

Morbidities associated with the use of metallic implants have stimulated interest in the development of resorbable ceramic implants that can provide structural support for a variety of clinical applications (including load-bearing and non load-bearing applications), and provide osteointegration over time. Resorbable bioceramic compositions filled with tricalcium phosphate (TCP), calcium sulfate, and other calcium phosphate salt-based bioceramics have previously been developed. These include resorbable bioceramic compositions derived from high modulus resorbable polymers such as PLLA (poly-l-lactic acid), PDLLA (poly-DL-lactic acid) and PLGA (polylactic-co-glycolic acid) that have been filled with TCP in order to improve osteointegration of the implant, and to tailor the resorption rate of the implant. Typically, these implants are limited to 30 vol-% or less of TCP in the composition.

In order to further improve the osteointegration of resorbable bioceramic filled implants it would be desirable to identify degradable polymers that can be filled with bioceramics at higher levels. It would also be desirable to identify bioceramic filled implants incorporating higher levels of bioceramics to provide a range of compositions such that the resorption rate of the implant can be tailored to the tissue healing. In addition, it would be desirable to identify degradable polymers that can be filled with bioceramics that resorb faster than PLLA, are tougher and less brittle, and that do not break down to yield highly acidic metabolites *in vivo* that can cause inflammatory responses.

It is an object of the present invention to provide compositions of bioceramics with P4HB with enhanced osteointegration, enhanced mechanical properties, and controlled degradation profiles, that can be used in medical applications.

It is another object of the present invention to provide methods for manufacturing biocompatible implants derived from resorbable bioceramics with P4HB.

It is still another object of the present invention to provide devices manufactured from bioceramic compositions with P4HB.

### SUMMARY OF THE INVENTION

The scope of the invention is defined by the claims.

Compositions for making implants comprising high levels of resorbable bioceramics have been developed. These compositions comprise P4HB filled with bioceramics. A preferred embodiment comprises P4HB filled with TCP.

### BRIEF DESCRIPTION OF THE DRAWINGS

Figure 1 is a diagram of pathways leading to the biosynthesis of poly-4-hydroxybutyrate. Pathway enzymes are: 1. Succinic semialdehyde dehydrogenase, 2. 4-hydroxybutyrate dehydrogenase, 3. diol oxidoreductase, 4. aldehyde dehydrogenase, 5. Coenzyme A transferase and 6. PHA synthetase.
Figure 2 is a prospective view of a tack made with the P4HB-bioceramic filled material of example 3.

### DETAILED DESCRIPTION OF THE INVENTION

### Definitions

"Poly-4-hydroxybutyrate" as generally used herein means a homopolymer comprising 4-hydroxybutyrate units, produced by a transgenic fermentation process, having a weight average molecular weight between 1,000 and 800,000 Da. It may be referred to herein as P4HB or TephaFLEX® biomaterial (manufactured by Tepha, Inc., Lexington, MA).

"Bioactive agent" is used herein to refer to therapeutic, prophylactic, and/or diagnostic agents. A biologically active agent is a substance used for, for example, the treatment, prevention, diagnosis, cure, or mitigation of a disease or disorder, a substance which affects the structure or function of the body, or pro-drugs, which become biologically active or more active after they have been placed in a predetermined physiological environment. Bioactive agents include biologically, physiologically, or pharmacologically active substances that act locally or systemically in the human or animal body. Examples can include, but are not limited to, small-molecule drugs, peptides, proteins, antibodies, sugars, polysaccharides, nucleotides, oligonucleotides, hyaluronic acid and derivatives thereof, aptamers, siRNA, nucleic acids, and combinations thereof. "Bioactive agent" includes a single such agent and is also intended to include a plurality.

"Bioceramic" means a ceramic suitable for use or replacement in the human body.

"Biocompatible" as generally used herein means the biological response to the material or device being appropriate for the device's intended application *in vivo.* Any metabolites of these materials should also be biocompatible.

"Blend" as generally used herein means a physical combination of different polymers, as opposed to a copolymer comprised of two or more different monomers.

"Ceramic" means an inorganic, nonmetallic solid prepared by the action of heat and subsequent cooling.

"Molecular weight" as used herein, unless otherwise specified, refers to the weight average molecular weight (Mw), not the number average molecular weight (Mn), and is measured by GPC relative to polystyrene.

"Resorbable" as generally used herein means the material is broken down in the body and eventually eliminated from the body.

"Resorbable bioceramic" means a bioceramic that is used to replace or repair damaged tissue in the body, and is eventually resorbed such that the host replaces the implant. Examples include tricalcium phosphate (TCP), biphasic calcium phosphate (BCP), hydroxylapatite, calcium sulfate, calcium carbonate, and other calcium phosphate salt-based bioceramics, including bioactive glasses composed of SiO₂, Na₂O, CaO and P₂O₅ in specific proportions.

### I. Compositions

Methods have been developed to produce bioceramic compositions comprising P4HB containing resorbable bioceramics having a size distribution between 0.1 and 500 microns at loadings up to 72% by weight and 50% by volume. These bioceramic compositions may be processed into biocompatible implants.

### A. P4HB Polymer and Copolymers

The processes described herein can typically be used with poly-4-hydroxybutyrate (P4HB). P4HB can be obtained from Tepha, Inc. of Lexington, MA. The polymer may comprise P4HB blended with other absorbable polymers such as homopolymers or copolymers of glycolic acid, lactic acid, *p*-dioxanone, trimethylene carbonate, ε-caprolactone or copolymers containing 4HB.

The P4HB polymers have a weight average molecular weight range from 1,000 to 800,000 Da.

In a preferred embodiment, the starting P4HB homopolymer is compounded with the bioceramic by metering in the desired ratio into a single or twin screw extruder wherein they are mixed prior to being extruded into pellets. These pellets can then be used to produce medical devices by existing processes used for thermoplastic polymers such as molding or extrusion.

### B. Resorbable Bioceramics

Resorbable bioceramics that can be used in the processes described herein must be: (i) biocompatible, (ii) eventually be resorbed by the body, and (iii) permit the replacement or repair of damaged tissues in the body. Examples of resorbable bioceramics include tricalcium phosphate (α and β forms of tricalcium phosphate (TCP) - with a nominal composition of Ca₃(PO₄)₂), biphasic calcium phosphate (BCP), hydroxylapatite, calcium sulfate, calcium carbonate, and other calcium phosphate salt-based bioceramics. Bio-active glasses may also be used. Bioactive glasses include bioactive glasses composed of SiO₂, Na₂O, CaO and P₂O₅ in specific proportions. The choice of bioceramic and particle size of the bioceramic will depend in part on the desired rate of resorption for the implant. In a preferred embodiment, P4HB polymer is filled with β-TCP, α-TCP or a combination thereof with a nominal particle size of 20 microns. The present invention relates to a medical device as defined by the claims comprising a biocompatible composition comprising up to 72% by weight or 50% by volume of the composition of a resorbable bioceramic having a size distribution between 0.1 and 500 microns.

P4HB polymers may also be blended with other polymers or materials to improve polymer properties, and filled with resorbable bioceramics. The P4HB polymer filled with bioceramics may also contain other additives including contrast agents, radiopaque markers or radioactive substances.

### II. Methods of Manufacturing P4HB Polymer Devices Filled with Resorbable Bioceramics

### A. Compounding of P4HB Polymer

Compositions of P4HB polymers filled with resorbable bioceramics can be prepared by compounding using a single or twin screw extruder. Alternatively, the P4HB polymer may be dissolved in a solvent, the bioceramic is then dispersed in the solvent solution, and the solvent removed by evaporation. Preferred solvents include acetone and chlorinated solvents such as methylene chloride and chloroform.

The P4HB formulations comprise up to 70% by weight or 50% by volume of the composition.

### B. Processing of Composition into Medical Devices

The P4HB polymer filled with resorbable bioceramics may be melt-processed into medical devices. In a preferred embodiment the devices may be injection molded or extruded.

The P4HB polymer compositions filled with resorbable bioceramic have tensile or compressive modulus values higher than for the polymers alone. A particular advantage of using P4HB polymer is the ability to prepare compositions with high percentages of bioceramic filler that are not brittle. In contrast to other degradable polymers such as PLLA, poly-3-hydroxybutyrate (P3HB, also denoted PHB), and polyhydroxybutyrate-co-valerate (PHBV), which are relatively brittle materials, P4HB polymer can help to toughen the resulting bioceramic composition. This means that at the same loading of bioceramic, compositions prepared with P4HB are less brittle than those prepared, for example, with PLLA. Improved toughness of an implant is particularly important to prevent breakage of the implant either during implantation or prior to the conclusion of healing.

Implants made from P4HB polymer filled with resorbable bioceramics have substantially improved properties for many medical applications relative to the same compositions made from brittle degradable thermoplastics.

If desired, implants made from P4HB polymer compositions filled with resorbable bioceramics may incorporate bioactive agents. These may be added during the formulation process, during the processing into molded parts or by coating/impregnating implants.

Implants made from P4HB polymer compositions filled with up to 72% by weight or 50% by volume of resorbable bioceramics may be used in the following medical devices: suture anchors, screws, pins, bone plates, interference screws, tacks, fasteners, rivets, staples, tissue engineering scaffolds, rotator cuff repair device, meniscus repair device, guided tissue repair/regeneration device, articular cartilage repair device, tendon repair device, ligament repair device, fixation device for an implant, fixation device for plastic surgery devices (including devices for fixation of facial and breast cosmetic and reconstructive devices), fixation device for a surgical mesh, facial reconstructive device, spinal fusion devices, device for treatment of osteoarthritis, imaging devices, and bone graft substitute.

Method of manufacturing are demonstrated by reference to the following non-limiting examples.

### Example 1: Compounding of P4HB and β-TCP

P4HB (Mw 350 kDa) was compounded with β-TCP using a Leistritz twin screw extruder with β-TCP loadings on a weight basis (wt-%) of 8.5%, 38%, and 69% (corresponding to loadings on a volume basis, (vol-%) of 3.4%, 19%, and 45%).

The β-TCP had a mean particle size of 20 ± 5 microns with 98% of the particles with a diameter of less than 75 microns. It conformed to ASTM F1088 with a purity, as measured by x-ray diffraction of >99%. The barrel temperature of the extruder increased from 100°C at the feed zone to 190°C at the die. The screws were rotated at 135rpm. The extruded strands were cooled in a water bath before being pelletized. The ash contents of the compounded compositions, including pure P4HB, are shown in Table 1.

**Table 1: Ash contents of P4HB compounded with β-TCP**

| Nominal wt-% β-TCP | P4HB Actual | | β-TCP - Actual | |
|---|---|---|---|---|
| | wt-% | vol-% | wt-% | vol-% |
| 0 | 100 | 100 | 0 | 0 |
| 10 | 91.5 | 96.6 | 8.5 | 3.4 |
| 40 | 62 | 81 | 38 | 19 |
| 72 | 31 | 55 | 69 | 45 |

### Example 2: Injection molding of P4HB compounded with β-TCP

Two inch dog bone test pieces were injection molded using an Arburg model 221 injection molder from the four samples shown in Table 1 of Example 1 after the samples were dried in a vacuum oven at room temperature for 48 hours. The barrel temperature increased from 170°C at the feed zone to 200°C at the end of the barrel. The mold temperature was maintained at 32°C. Dog bone samples for each composition were tested for tensile properties in at least triplicate using an MTS test machine with a 2 inch/min cross head speed.

The tensile properties for each composition are shown in Table 2. Notably, the modulus of the compounded composition increases as the percentage of β-TCP in the composition increases.

**Table 2: Tensile test results for dog bones of P4HB filled at different levels with β-TCP**

| | vol % | Wt % | Modulus (psi) | Yield Stress (psi) | Strain at Yield (%) | Break Stress (psi) | Strain at Break (%) |
|---|---|---|---|---|---|---|---|
| PHA Extruded | 0 | 0 | 48,600 | 3,070 | 15 | 5,200 | 230 |
| PHA 4 | 3.4 | 8.5 | 54,000 | 3,170 | 12 | 4,800 | 190 |
| PHA 20 | 19 | 38 | 81,900 | 3,000 | 14 | 3,300 | 92 |
| PHA 50 | 45 | 69 | 193,500 | 2,170 | 1 | | 4 |

[in table 2, 1 psi corresponds to 6894.7572931783 Pa]

### Example 3: Injection molding of interference screws of P4HB compounded with β-TCP

P4HB was compounded with β-TCP to provide a composition with 53 wt% β-TCP.

The intrinsic viscosity of the formulation prior to injection molding was 1.79 dL/g. Interference screws with a diameter of 7mm and length of 20mm were injection molded. After injection molding of the screws the intrinsic viscosity of the composition was essentially identical, indicating little loss of molecular weight during the injection molding process. For comparative testing, screws of the same design were molded from the P4HB alone.

The torsional strength of the screws was determined by embedding the tip of the screw in epoxy resin and measuring the maximum torque achieved by the screwdriver before failure. For the biocomposite screw, the average of three screws tested gave a value of 14.0 Ncm. For the P4HB screw, the average of three tests gave 7.3 Ncm. For comparison, an Arthrex Biointerference screw composed of PLLA was also tested. This gave an average failure torque of 12.1Ncm.

### Example 4: Compounding of P4HB with Calcium Carbonate (comparative example, not forming part of the invention)

A DSM Xplore™ 15cm³ Twin Screw Microcompounder was used to compound P4HB with 44 weight % of Calcium Carbonate at a temperature of 220°C. The Calcium Carbonate had a nominal particle size of 10 microns.

The rod of material extruded from the Microcompounder was collected and tensile testing showed it to have a modulus of 130MPa and a strain at failure of 239% demonstrating that the compounded material was ductile.

### Example 5: Tack for Attachment of Plastic Surgery Mesh

Rods of compounded material from Example 3 were produced at the same time as the injection molding of the interference screws. These rods were subsequently machined using a lathe to produce tacks. The design and dimensions (in mm) of the tacks are shown in Figure 2.

Holes were produced in surrogate bone and cow bone using drills and awls and it was shown that the tack could be used to hold a resorbable mesh in place. Tacks of this design were also produced from PHA material without ceramic filler. Tacks of this design are suitable for use in plastic surgery procedures such as brow lifts to attach mesh to the skull.

### Example 6: Analysis of TCP Distribution in a P4HB/TCP Blend and P4HB/TCP Device

The distribution of TCP in a P4HB/TCP pellet and a P4HB/TCP pin was characterized by energy dispersive spectroscopy (EDS). Each test sample was embedded in paraffin and cross-sectioned using a microtome. Cross sections in the transverse and longitudinal directions were obtained from each article. The test articles were analyzed by EDS to verify the absence of foreign material, and to map the locations of TCP particles in the polymer medium. Backscatter electron micrographs at 75X and 500X magnification were collected from each test article to examine the general distribution pattern of the TCP particles. The TCP particles of both test samples were found to be evenly distributed through the polymer medium.

### Example 7: Assessment of Local Tissue Reaction to P4HB/TCP Pins in a Rabbit Tibial Defect Model and Retention of Shear Strength and Molecular Weight Loss in a Subcutaneous Pocket

The purpose of this study was to test the local response in bone to an implanted P4HB/TCP pin, and additionally the strength retention and molecular weight loss of the P4HB/TCP pins implanted subcutaneously. The test articles were 2 x 70 mm P4HB/TCP pins. For the bone implantation, Orthosorb® (poly-p-dioxanone (PDS))Resorbable Pins measuring 2 x 40 mm were used as controls.

Two bilaterial drill defects were created in the tibia and filled with the test article (n=10) on one side, and a control article (n=10) on the other. The test and control articles were cylindrical implants approximately 2 mm in diameter and 6 mm in length. In addition, each animal had two rods, approximately 2 x 35 mm, of the test material implanted into separate subcutaneous pockets on the dorsal back that were retrieved at necropsy.

At necropsy, after an in-life period of 4 weeks, the tibial defect sites were excised, placed in formalin, and processed for standard histopathological analysis. One section was prepared from each implant, each stained by hemotoxylin/eosin (H&E). Each site was analyzed by a pathologist for local tissue reaction and any signs of bone development and ingrowth. The subcutaneously implanted test article was evaluated macroscopically for capsule formation or other signs of irritation and then tested for molecular weight retention by GPC relative to polystyrene, and shear strength.

The P4HB/TCP pins in the rabbit tibial drill model to assess local tissue reaction to bone implants at 4 weeks were found to be non-irritants when compared to the control article. The subcutaneously implanted P4HB/TCP pins were found to have retained 92% of their shear strength and 87% of their original weight average molecular weight after 4 weeks *in vivo.*

## Claims

1. A medical device comprising a biocompatible composition comprising
a poly-4-hydroxybutyrate (P4HB) homopolymer produced by a transgenic fermentation process, having a weight average molecular weight between 1,000 and 800,000 Da, and
up to 72% by weight or 50% by volume of the composition of a resorbable bioceramic having a size distribution between 0.1 and 500 microns, and
having a tensile modulus of >0.34 GPa (>50,000 psi),
wherein the device is selected from the group consisting of a suture anchor, screw, pin, bone plate, interference screw, tack, fastener, rivets, staples, tissue engineering scaffold, rotator cuff repair device, meniscus repair device, guided tissue repair/regeneration device, articular cartilage repair device, tendon repair device, ligament repair device, fixation device for an implant, fixation device for a plastic surgery device including facial and breast cosmetic and reconstructive devices, fixation device for a surgical mesh, facial reconstructive device, spinal fusion device, device for treatment of osteoarthritis, imaging device, and bone graft substitute.

2. The device of claim 1 wherein the bioceramic is α-tricalcium phosphate (TCP), β-TCP, a combination of α- and β-TCP, calcium sulfate, calcium carbonate, or a calcium phosphate salt-based bioceramic.

3. The device of claim 1 wherein the composition comprises a blend of one or more polymers with the P4HB homopolymer.

4. The device of claim 3 wherein the polymers are resorbable, and optionally wherein the one or more polymers are derived from glycolic acid, glycolide, lactic acid, lactide, p-dioxanone, trimethylene carbonate, or ε-caprolactone monomers.

5. The device of claim 1 wherein the device is formed by injection molding of the composition, extrusion of the composition, or by machining a molded form of the composition.

6. The device of claim 1 wherein the fixation device is suitable for use in fixing surgical mesh to bone, or for a brow-lift.

7. The device of claim 1 wherein the device further comprises a bioactive agent, contrast agent, radiopaque marker and/or a radioactive substance.

8. The device of claim 7 wherein the bioactive agent is a small-molecule drug, peptide, protein, antibody, sugar, polysaccharide, nucleotide, oligonucleotide, hyaluronic acid or derivatives thereof, aptamer, siRNA, or nucleic acid.

9. The device of claim 1 wherein the device is formed by extrusion of the composition or by machining an extruded form of the composition.

10. The device of claim 1, wherein the biocompatible composition is derived by compounding P4HB with calcium carbonate at a temperature of 220°C.

11. A method of preparing the biocompatible device of claim 1, comprising: (a) providing powder or pellets of the poly-4-hydroxybutyrate homopolymer and the bioceramic, heating to a melt temperature in the range of 150 to 300°C, extruding the melted composition and forming the device; (b) injection molding at a melt temperature in the range of 150 to 300°C, the P4HB homopolymer and the bioceramic, and forming the device; (c) solvent blending the P4HB homopolymer with the bioceramic and forming the device; or (d) machining a molded rod of the composition to produce the required device design.

## Patentansprüche

1. Medizinvorrichtung, die eine biokompatible Zusammensetzung umfasst, die ein Poly-4-hydroxybutyrat (P4HB)-Homopolymer umfasst, das durch ein transgenes Fermentationsverfahren erzeugt ist, mit einem Gewichtsmittel des Molekulargewichts zwischen 1.000 und 800.000 Da und bis zu 72 Gew.-% oder 50 Gew.-% Volumen der Zusammensetzung einer resorbierbaren Biokeramik mit einer Größenverteilung zwischen 0,1 und 500 Mikrometern und mit einem Zugmodul von > 0,34 GPa (> 50.000 psi), wobei die Vorrichtung ausgewählt ist, aus der Gruppe bestehend aus einem Nahtanker, einer Schraube, einem Stift, einer Knochenplatte, einer Interferenzschraube, einem Nagel, einem Befestigungselement, Nieten, Heftklammern, einem Gerüst zur Gewebezüchtung, eine Rotatorenmanschettenreparaturvorrichtung, einer Meniskusreparaturvorrichtung, einer geführten Vorrichtung zur Gewebereparatur/-regeneration, einer Gelenkknorpelreparaturvorrichtung, einer Sehnenreparaturvorrichtung, einer Bänderreparaturvorrichtung, einer Fixierungsvorrichtung für ein Implantat, einer Fixierungsvorrichtung für eine plastische Chirurgievorrichtung, die Gesichts- und Brustkosmetik und rekonstruktive Vorrichtungen umfasst, eine Fixierungsvorrichtung für ein chirurgisches Netz, eine Gesichtsrekonstruktionsvorrichtung, eine Wirbelsäulenfusionsvorrichtung, eine Vorrichtung zur Behandlung von Osteoarthritis, eine Bildgebungsvorrichtung und Knochenersatzmaterial.

2. Vorrichtung nach Anspruch 1, wobei die Biokeramik α-Tricalciumphosphat (TCP), β-TCP, eine Kombination aus α- und β-TCP, Calciumsulfat, Calciumcarbonat oder eine auf Calciumphosphatsalz basierende Biokeramik ist.

3. Vorrichtung nach Anspruch 1, wobei die Zusammensetzung eine Mischung aus einem oder mehreren Polymeren mit dem P4HB-Homopolymer umfasst.

4. Vorrichtung nach Anspruch 3, wobei die Polymere resorbierbar sind, und wahlweise wobei die ein oder mehreren Polymere abgeleitet sind von Glykolsäure, Glycolid, Milchsäure, Lactid, p-Dioxanon, Trimethylencarbonat oder ε-Caprolacton-Monomeren.

5. Vorrichtung nach Anspruch 1, wobei die Vorrichtung durch Spritzgießen der Zusammensetzung, Extrudieren der Zusammensetzung, oder durch maschinelle Bearbeitung einer gebildeten Form der Zusammensetzung gebildet wird.

6. Vorrichtung nach Anspruch 1, wobei die Fixierungsvorrichtung zur Verwendung beim Befestigen eines chirurgischen Netzes an Knochen oder für einen Augenbrauenlift geeignet ist.

7. Vorrichtung nach Anspruch 1, wobei die Vorrichtung ferner ein bioaktives Mittel, ein Kontrastmittel, eine röntgendichte Markierung und/oder eine radioaktive Substanz umfasst.

8. Vorrichtung nach Anspruch 7, wobei das bioaktive Mittel ein niedermolekulares Arzneimittel, Peptid, Protein, Antikörper, Zucker, Polysaccharid, Nukleotid, Oligonukleotid, Hyaluronsäure oder Derivate davon, Aptamer, siRNA oder Nukleinsäure ist.

9. Vorrichtung nach Anspruch 1, wobei die Vorrichtung durch Extrusion der Zusammensetzung oder durch maschinelle Bearbeitung einer extrudierten Form der Zusammensetzung gebildet wird.

10. Vorrichtung nach Anspruch 1, wobei die biokompatible Zusammensetzung durch Vermischen von P4HB mit Calciumcarbonat bei einer Temperatur von 220 °C abgeleitet wird.

11. Verfahren zur Vorbereitung der biokompatiblen Vorrichtung nach Anspruch 1, umfassend:
(a) Bereitstellen von Pulver oder Pellets aus dem Poly-4-hydroxybutyrat-Homopolymer und der Biokeramik, Erwärmen auf eine Schmelztemperatur im Bereich von 150 bis 300 °C, Extrudieren der geschmolzenen Zusammensetzung und Formen der Vorrichtung;
(b) Spritzgießen des P4HB-Homopolymers und der Biokeramik bei einer Schmelztemperatur im Bereich von 150 bis 300 °C und Formen der Vorrichtung;
(c) Lösungsmittelvermischen des P4HB-Homopolymers mit der Biokeramik und Formen der Vorrichtung; oder
(d) Bearbeiten eines geformten Stabs der Zusammensetzung, um das erforderliche Vorrichtungsdesign herzustellen.

## Revendications

1. Dispositif médical comprenant une composition biocompatible comprenant un homopolymère de poly-4-hydroxybutyrate (P4HB) produit par un procédé de fermentation transgénique, ayant un poids moléculaire moyen compris entre 1 000 et 800 000 Da et allant jusqu'à 72 % en poids ou 50 % en volume de la composition d'une biocéramique résorbable ayant une distribution de taille comprise entre 0,1 et 500 microns et un module de traction supérieur à 0,34 GPa (>50 000 psi), dans laquelle le dispositif est choisi dans le groupe comprenant une ancre de suture, une vis, une goupille, une plaque osseuse, une vis d'interférence, un clou, une attache, des rivets, des agrafes, un échafaudage d'ingénierie tissulaire, un dispositif de réparation de coiffe des rotateurs, un dispositif de réparation du ménisque, un dispositif guidé de réparation/régénération des tissus, un dispositif de réparation du cartilage articulaire, un dispositif de réparation du tendon, un dispositif de réparation du ligament, un dispositif de fixation pour un implant, un dispositif de fixation pour un dispositif de chirurgie plastique comprenant des dispositifs cosmétiques et de reconstruction du visage et du sein, un dispositif de fixation pour un treillis chirurgical, un dispositif de reconstruction du visage, un dispositif de fusion de la colonne vertébrale, un dispositif de traitement de l'ostéoarthrite, un dispositif d'imagerie et un substitut de greffe osseuse.

2. Dispositif selon la revendication 1, dans laquelle la biocéramique est le phosphate α-tricalcique (TCP), β-TCP, une combinaison de α- et β-TCP, le sulfate de calcium, le carbonate de calcium ou une biocéramique à base de sel de phosphate de calcium.

3. Dispositif selon la revendication 1, dans laquelle la composition comprend un mélange d'un ou plusieurs polymères avec l'homopolymère P4HB.

4. Dispositif selon la revendication 3, dans laquelle les polymères sont résorbables, et éventuellement dans lequel le ou les polymères sont dérivés de monomères d'acide glycolique, de glycolide, d'acide lactique, de lactide, de p-dioxanone, de triméthylène carbonate ou d'ε-caprolactone.

5. Dispositif selon la revendication 1, dans laquelle le dispositif est formé par moulage par injection de la composition, de l'extrusion de la composition ou par usinage d'une forme moulée de la composition.

6. Dispositif selon la revendication 1, dans laquelle le dispositif de fixation est approprié pour être utilisé dans la fixation d'un treillis chirurgical à un os, ou pour un lifting frontal.

7. Dispositif selon la revendication 1, dans laquelle le dispositif comprend en outre un agent bioactif, un agent de contraste, un marqueur radio-opaque et/ou une substance radioactive.

8. Dispositif selon la revendication 7, dans laquelle l'agent bioactif est un médicament à petite molécule, peptide, protéine, anticorps, sucre, polysaccharide, nucléotide, oligonucléotide, acide hyaluronique ou ses dérivés, aptamère, petit ARN interférent ou acide nucléique.

9. Dispositif selon la revendication 1, dans laquelle le dispositif est formé par extrusion de la composition ou par usinage d'une forme extrudée de la composition.

10. Dispositif selon la revendication 1, dans laquelle la composition biocompatible est dérivée en mélangeant P4HB avec du carbonate de calcium à une température de 220 °C.

11. Procédé de préparation du dispositif biocompatible selon la revendication 1, comprenant les étapes suivantes :
(a) fournir une poudre ou des pastilles de l'homopolymère poly-4-hydroxybutyrate et de la biocéramique, chauffer à une température de fusion comprise entre 150 et 300 °C, extruder la composition fondue et former le dispositif ;
(b) mouler par injection à une température de fusion comprise entre 150 et 300 °C, l'homopolymère P4HB et la biocéramique, et former le dispositif ;
(c) mélanger au solvant l'homopolymère P4HB avec la biocéramique et former le dispositif ; ou
(d) usiner une tige moulée de la composition pour produire la conception de dispositif requise.
